Europäisches Patentamt

European Patent Office

Office européen des brevets

(19)

(11) Publication number: **0 251 471**
A1

# (12) EUROPEAN PATENT APPLICATION

(21) Application number: 87304457.2

(22) Date of filing: 19.05.87

(51) Int. Cl.³: **A 61 M 25/00**

(30) Priority: 02.06.86 US 872680

(43) Date of publication of application:
07.01.88 Bulletin 88/1

(84) Designated Contracting States:
AT BE CH DE ES FR GB GR IT LI LU NL SE

(71) Applicant: Vaillancourt, Vincent L.
30A Ridgedale Avenue
East Hanover New Jersey 07939(US)

(72) Inventor: Vaillancourt, Vincent L.
14,Bunyan Drive
Livingstone,New Jersey 07038(US)

(72) Inventor: Timothy, Earle J.
8 Valley Road
Clinton,Connecticut 06413(US)

(74) Representative: Silverman, Warren et al,
HASELTINE LAKE & CO. Hazlitt House 28 Southampton
Buildings Chancery Lane
London WC2A 1AT(GB)

(54) Naso-gastric intubation system.

(57) A naso-gastric tube (10) or an adapter for an intubation
tube is coated in a transparent section (16) with a pH sensitive
coating (14) which changes color in response to the passage
of gastric fluids from the stomach of a patient (13). Initially,
the coating (14) may be made with a red color provided by
Congo Red which, upon passage of gastric fluids, changes to
a blue color. The naso-gastric tube (10) can be dipped into a
solution containing a pH indicator and thereafter dried and
cured so as to bond the coating (14) within the tube (10).

Fig.2

Fig. I

EP 0 251 471 A1

# NASO-GASTRIC INTUBATION SYSTEM

This invention relates to a naso-gastric intubation system having a pH indicator. More particularly, this invention relates to a pH sensitive coating for an intubation system.

Heretofore, various types of systems have been known for removing fluids from or feeding fluids to the gastrointestinal track of a patient. Generally, such systems employ tubes of the naso-gastric type or of the oral type. In the first case, the tube is passed through the nasal passage and esophagus into the stomach of a patient and, in some cases, into the duodenum. In many cases, it is necessary to position the distal end of the tube at an exact location within the stomach or the duodenum in order to remove or feed in fluids. In the past, various techniques have been used to determine the position of the distal end of the tube within the patient.

For example, where the tubes have been provided with apertures at the distal end, stethoscopes have been used to detect the gurgling of fluid through the apertures of the tube and, thus, provide an indication of the distal end of the tube. It has also been suggested that air be injected through the tube into the stomach of a patient for detection by a stethoscope. In other cases, it has been proposed to determine the position of the distal end of a tube by X-ray or fluoroscopic examination, provided the tube has been rendered radiopaque through the addition of a suitable material in the tube. Other suggested techniques include the use of markers on the tube to indicate the depth of penetration and the extraction of specimens of fluid in order to verify by appearance that the fluid, for example, is a gastric fluid. Such techniques, however, are cumbersome to use, expensive where X-ray or fluoroscopic examination is required, and in some cases not particularly accurate or reliable.

U.S. Patent 4,381,011 describes an internal feeding apparatus which employs an electrode at the distal end of a tube as a pH measuring means since the fluids within the esophagus, stomach and duodenum have different acidities. In this case, the electrode is connected via electrical leads to a pH monitoring device located at a remote point from a patient. However, such a construction is rather expensive, particularly, in cases where the tubes are intended for a single use and are therefore made to be disposable.

Accordingly, it is an object of the invention to provide a relatively simple technique to determine the position of a naso-gastric tube within the patient.

Briefly, the invention provides a pH sensitive coating for use with an intubation system which is characterized in being able to change color in response to a flow of gastric fluid. thereover.

In one embodiment, the invention provides an intubation system which is comprised of a means for defining a flow path for a flow of gastric fluids with the pH sensitive coating within the flow path. The means for defining the flow path may include a flexible elongated naso-gastric tube. In one case, the coating is disposed in the proximal end of the tube while in another case, the means includes an adapter between the tube and a means for drawing gastric fluid into and through the tube from a distal end thereof. In this case, the pH sensitive coating is disposed in the adapter.

The invention also provides an adapter for an intubation system which is comprised of a transparent tube which defines a flow path for a flow of gastric fluids and a pH sensitive coating within the tube. In this case, the tube includes a distal end for fitting into an intubation tube and a proximal end for receiving a means for aspirating the intubation tube.

The naso-gastric tube is made of any suitable material such as polyurethane which is commonly used for the enteric feeding of a patient. In this regard, the tube is of flexible.

- 4 -

nature and has one or more openings at a distal end for the entry of gastric fluids into the tube and for the feeding of suitable fluids into a patient, for example, into the stomach of a patient.

The pH sensitive coating can be made, for example of a hydroxy-terminated hydrophilic polyurethane having an average molecular weight of about 7500 dissolved in an ethyl alcohol solvent, for example as described in copending U.S. patent application Serial No. 781,218, filed September 25, 1985. In addition, the coating includes a pH sensitive indicator in an amount sufficient to render the coating sensitive to a flow of gastric fluid having a pH in the range of from 3.0 to 4.0 to change color. This pH sensitive indicator is selected, for example from the group consisting of Congo Red, Bromophenol Blue, Methyl Yellow, Chlorophenal Blue, Methyl Orange and Bromochlorophenol Blue. For example, where Congo Red is used as the pH indicator in the coating, the coating will turn from red to blue when contacted with gastric juices.

In a further embodiment, the intubation system may include a naso-gastric tube which is capable of selectively removing gastric fluids from a patient and delivering a nutrient solution to a patient as well as an administration set for feeding fluids into the tube. In this case, the administration set may include a Y-site with a branch line having the coating therein for removal of gastric fluids therethrough.

Where a naso-gastric tube is to be used to feed fluids into the stomach, the tube is initially passed through the nasal passage and esophagus of the patient into the stomach. At this time, the coating which is applied to a proximal end of the tube or within an adapter at the proximal end of the tube appears red. After placement of the tube and upon aspiration, for example, using a syringe, the stomach fluids pass through the tube and upon contacting the coating change the color of the coating, for example to blue. In this manner, the nurse or technician inserting the tube has visual confirmation that the tube is in the stomach. Thereafter, upon the introduction of fluids into the naso-gastric tube for feeding into the stomach, the coating color reverts to the original color, i.e. red, and verifies to the nurse that the coating changed color as a result of a significant pH shift.

In order to provide a naso-gastric tube with a coating, one end of the tube is placed into a coating solution containing a pH sensitive material for a time sufficient to coat an interior of the tube with the solution. The tube is then removed from the solution to effect drying of the tube and, thereafter, the solution is cured to form a bonded coating of the solution material within the tube.

These and other objects and advantages of the invention will become more apparent from the following detailed description taken in conjunction with the accompanying drawings wherein:

Fig. 1 illustrates a perspective view of a naso-gastric tube having a pH indicator and disposed in a patient;

Fig. 2 illustrates a part cross-sectional view of the proximal end of the naso-gastric tube coated in accordance with the invention;

Fig. 3 illustrates a cross-sectional view similar to Fig. 2 during passage of gastric fluid therethrough;

Fig. 4 illustrates a view of an administration set having a Y-site with a branch line provided with the pH sensitive coating; and

Fig. 5 illustrates an exploded view of an intubation system employing an adapter with a pH coating therein in accordance with the invention.

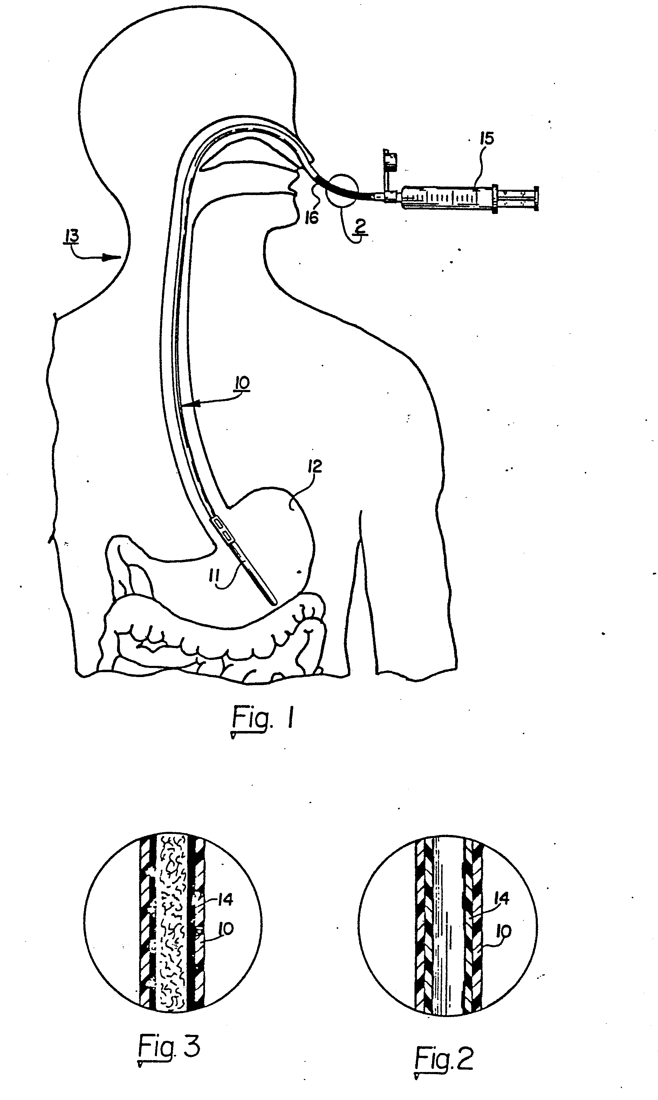

Referring to Fig. 1, the elongated naso-gastric tube 10 is made of any suitable material, such as polyurethane and is sized to define a flow path for a flow of gastric fluid. As indicated, the tube 10 has a transparent proximal end while the distal end is provided with one or more openings 11 for the introduction of fluids into the stomach 12 of a patient 13. In addition, as shown in Fig. 2, the tube 10 is provided with a pH sensitive coating 14 within the transparent proximal end of the tube. This coating 14 is characterized in being able to change color in response to a flow of gastric fluid thereover.

The coating 14 is made, for example of a hydroxy-terminated hydrophilic polyurethane having an average molecular weight of about 7500 dissolved in an ethyl alcohol solvent. For example, the coating solution may be made as described in copending U.S. patent application Serial No. 781,218, filed September 25, 1985. In addition, the coating solution includes a pH indicator which renders the coating sensitive to a pH in the range of from 3.0 to 4.0 in order to change color. The pH sensitive indicator is selected from the group consisting of Congo Red, Bromophenol Blue, Methyl Yellow, Chlorophenol Blue, Methyl Orange and Bromochlorophenol Blue.

Referring to Fig. 1, the naso-gastric tube 10 may be incorporated into a system which includes a means, in the form of a syringe 15 which can be connected to the proximal end of the tube 10 for drawing gastric fluids from the stomach 12 of the patient 13 into and through the tube 10 via the openings 11. As this fluid passes over the coating 14 at the proximal end of the tube 10, the coating 14 changes color due to the acidic nature of the gastric fluids. In this respect, it is known that the gastric fluids of the stomach are at a pH of about 1.5. Where the coating contains, for example, Congo Red which imparts a red color to the coating, the gastric fluids change the coating color to blue.

Should the extracted fluids not change the color of the coating 14, such would be an indication that the tube 10 has not reached the proper position of the stomach 12. However,

- 8 -

once the tube 10 is positioned at the upper point of the stomach, the flow of gastric fluids will change the color of the coating 14. At that time, the tube can be connected to a suitable means such as an administration set for the introduction of the fluids into the patient 13.

In order to make a pH sensitive naso-gastric tube, one end of the elongated flexible tube 10 is placed into a coating solution containing the pH sensitive material for a time sufficient to coat an interior of the tube of the solution. For example, the tube 10 can be dipped into the coating solution for fifteen seconds. Thereafter, the tube is removed from the solution and air dried for ten minutes in order to effect drying of the tube 10. Next, the solution which is coated on the tube 10 is cured, for example in an air circulating oven at 100°C for ten minutes in order to form a bonded coating 14 of the solution material within the tube 10.

By way of example, in order to provide a red coating on the tube 10, 0.005 grams of Congo Red is dissolved in 40 cubic centimeters of the coating solution which is described above. This imparts a red color to the transparent portion of the tube 10. When the stomach gastric fluids pass over this coating, the color will change to blue as indicated in Fig. 2.

Alternatively, the coating solution may be formed of 20 cubic centimeters of the basic coating solution with two drops of Bromophenol Blue dissolved therein as the pH sensitive material.  In this case, the coating is normally blue or blue-green in color.  When subjected to the gastric fluids, the color will change to yellow.

The pH indicator may also be applied to a previously coated tube.  For example, a solution of pH indicator can be made into which a pre-lubricated tube 10 can be immersed. For example, a pH solution may be made by stirring 0.05 grams of Congo Red into 15 cubic centimeters of water until a solution is formed.  Thereafter, a tube which has been previously coated with the basic coating, for example, as described in copending application Serial No. 781,218 is dipped into the indicator solution, for example, to a depth of about 1 1/2 inches at room temperature and allowed to soak for ten minutes.  Thereafter, the tube can be removed from the indicator solution and dabbed into an absorbent material in order to prevent occlusion and also to remove excess indicator solution.  The tube may then be dried at room temperature for about thirty minutes.

When the coating is exposed to the gastric juices, the change in color will occur over a relatively short time, for example, less than five seconds.  Thus, the nurse inserting the tube 10 into the patient 13 can obtain a relatively rapid visual indication that the tube 10 has been correctly placed after the gastric fluids have been aspirated through the tube 10.

The following is a list of pH indicators which give a distinct color change for a pH range of from 3.0 to 4.0.

| INDICATOR | COLOR CHANGE | pH AT COLOR CHANGE |
|---|---|---|
| Methyl Yellow | Yellow to Red | 3.2 |
| Bromophenol Blue | Blue to Yellow | 3.5 |
| Chlorophenol | Purple to Yellow | 3.7 |
| Congo Red | Red to Blue | 3.5 |
| Methyl Orange | Yellow to Pink | 3.8 |
| Bromochlorophenol Blue | Blue to Yellow | 3.8 |

Providing the transparent proximal end of the naso-gastric tube 10 with a pH sensitive coating results in a relatively simple technique to indicate the position of the tube in the stomach of a patient. Further, there is no need for expensive detection devices in order to detect when the tube has reached the gastric fluid region of a patient's stomach. Further, because of its simplicity, the tube can be manufactured as a disposable item at relatively low costs.

The naso-gastric tube 10 may also be provided with a stylet wire, for example as described in copending application Serial No. 781,218 or any other suitable insertion device.

The coating solution may also be a hydrophilic polyurethane coating made from seventy-five parts methylene bichloride and twenty-five parts of ethyl alcohol solution. This solution may also include three-one half parts hydrophillic polyurethane polymer (described elsewhere in this specification) with five parts TRITON-X-100® (an alkyl phenoxy polyethoxy ethanol) to achieve rapid fluid uptake. Where Congo Red is used, the dye content is 87% and is added at the rate of 0.005 grams per 40 cubic centimeters of the solution.

0251471

In the case of Bromophenol Blue, two drops of a 0.04% solution of Bromophenol Blue is added to 20 cubic centimeters of the coating solution.

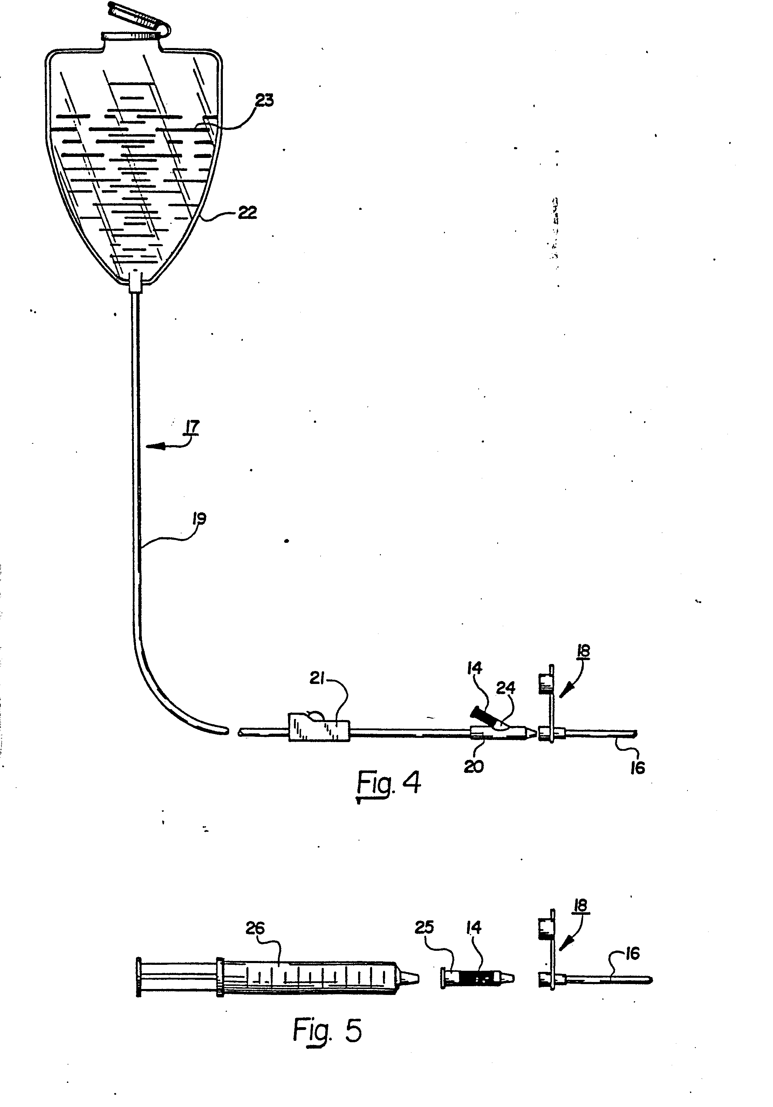

Referring to Fig. 4, the intubation system includes a naso-gastric tube 16 of conventional type and construction and an administration set 17 which can be selectively connected to the proximal end of the tube 16. As indicated, the tube 16 includes a closure means 18 at the proximal end for sealing the tube 16 when required. This means is of conventional construction and need not be further described.

The administration set 17 includes a delivery tube 19, a Y-site 20 at the distal end of the tube 19, a clamp 21 of conventional structure for closing off the flow of fluids through the tube 19 and a container 22 having a nutrient solution 23 therein for delivery through the tube 19 into the naso-gastric tube 16 and, thence, to a patient. The Y-site 20 also includes a branch 24 in which a pH coating as described above is disposed on the inside surface.

The naso-gastric tube 16 serves to selectively remove gastric fluids from a patient and for delivering the nutrient solution 23 to the patient. As such, the naso-gastric tube 16 and the administration set 17 define a flow path for a flow of gastric fluids from the patient.

In use, with the naso-gastric tube 16 introduced into a patient, a nurse may draw gastric fluids through the tube 16 and the branch line 24 of the Y-site of the administration set 17, for example using a syringe (not shown). At this time, the pH sensitive coating is available to sense whether or not the naso-gastric tube 16 is in proper position. If so, the syringe can be removed and the clamp 21 may be opened so as to begin delivery of the nutrient solution 23 to the patient.

Referring to Fig. 5, wherein like reference characters indicate like parts as above, the intubation system may employ a naso-gastric tube 16 and an adapter 25 at the proximal end of the tube 16. In this embodiment, the pH sensitive coating is disposed within the adapter 25. As above, a syringe 26 is provided for aspirating the tube 16 in order to draw gastric fluids through the tube 16 into and through the adapter 25 for testing by the pH sensitive coating 14.

In this latter embodiment, after the naso-gastric tube 16 has been positioned in place, the adapter 25 can be removed and disposed of in a suitable manner. Since the adapter may be of relatively small size for the purposes intended, the overall cost of making an adapter with a pH sensitive coating can be reduced to a minimum. The adapter 25 may be made of any suitable materials and is provided with a transparent section through which the color of the pH sensitive coating can be readily viewed.

The invention thus provides a simple technique of providing a pH sensitive indicator for use in an intubation system in a relatively simple manner and in a manner which quickly shows by a color change, the passage of gastric fluids from the stomach. In this respect, since the fluids in the esophagus are not as acidic as the gastric fluids, the pH sensitive material is selected so as to change color for the pH range to be expected of the stomach gastric fluids.

Claims:

1. An intubation system comprising a flexible tube (10, 16) defining a flow path for a flow of gastric fluids characterized in having a pH sensitive coating (14) within said flow path capable of changing color in response to a flow of gastric fluid thereover.

2. An intubation system as set forth in claim 1, further characterized in having an adapter (25) at a proximal end of said tube for drawing gastric fluid into and through said tube (10, 16) from a distal end thereof with said coating (14) being disposed in said adapter (25).

3. An intubation system as set forth in claim 1, further characterized in having an administration set (17) for feeding fluids into said tube (16), said administration set (17) including a Y-site (20) with a branch line (24) having said coating (14) therein for removal of gastric fluids therethrough.

4. An intubation system as set forth in claim 1, 2 or 3, characterized in that said coating (14) is made of a hydroxy-terminated hydrophilic polyurethane having an average molecular weight of about 7500 dissolved in an ethyl alcohol solvent.

5. An intubation system as set forth in any of claims 1 to 4 characterized in that said coating is sensitive to a pH in the range of from 3.0 to 4.0 to change color.

6. An intubation system as set forth in any of claims 1 to 4 characterized in that said coating includes a pH sensitive indicator selected from the group consisting of Congo Red, Bromophenol Blue, Methyl Yellow, Chlorophenol Blue, Methyl Orange and Bromochlorophenol Blue.

7. A method of making a pH sensitive intubation system comprising the steps of placing one end of a tube (10) in a coating solution for a time sufficient to coat an interior of the tube with the solution; removing the tube (10) from the solution to effect drying of the tube; and thereafter curing the solution coated on the tube to form a bonded coating (14) of the solution material within the tube (10) ·characterized in that the coating solution contains a pH sensitive material capable of changing color in response to a flow of gastric fluid.

8. A method as set forth in claim 7 characterized in that the coating solution is made of a hydroxy-terminated hydrophilic polyurethane having an average molecular weight of about 7500 dissolved in an ethyl alcohol solvent.

9. A method of determining the position of an intubation tube in the stomach of a patient, said method including the steps of introducing an intubation tube (10) into the stomach of a patient and, aspirating a flow of gastric-fluids from the stomach through the tube characterized in passing the flow of gastric fluid over a pH sensitive coating (14) which is able to change color in response to a flow of gastric-fluid in a pH range of from 3.0 to 4.0; and stopping the introduction of the tube (10) into the stomach of the patient in response to said

- 16 -

coating (14) changing color.

10. A method as set forth in claim 9 characterized in that the pH sensitive coating is disposed in a proximal transparent end of the tube.

-7-

_Fig. 1_

_Fig. 3_

_Fig. 2_

Fig. 4

Fig. 5

**0251471**

Application number

# PARTIAL EUROPEAN SEARCH REPORT

European Patent Office

which under Rule 45 of the European Patent Convention shall be considered, for the purposes of subsequent proceedings, as the European search report

| DOCUMENTS CONSIDERED TO BE RELEVANT | | | EP 87304457.2 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | US - A - 3 373 735 (J.P. GALLAGHER) | 1,5,6 | A 61 M 25/00 |
| A | * Totality; especially column 2, table 1 * | 2 | |
| | -- | | |
| A | EP - A2 - 0 073 558 (S.R. GOLDSTEIN et al.) | 1 | |
| | * Totality; especially fig. 1; page 5, paragraph 1 * | | |
| | -- | | |
| A | US - A - 4 200 110 (J.I. PETERSON et al.) | 1,7 | |
| | * Totality; especially fig. 1; column 1, line 67 - column 2, line 13; column 3, line 58 - column 4, line 9 * | | |
| | -- | | |
| D,A | US - A - 4 381 011 (L.S. SOMERS) | | TECHNICAL FIELDS SEARCHED (Int. Cl.4) |
| | * Totality * | | |
| | -- | | A 61 B 5/00 |
| | | | A 61 L 29/00 |

## INCOMPLETE SEARCH

A 61 M 25/00

The Search Division considers that the present European patent application does not comply with the provisions of the European Patent Convention to such an extent that it is not possible to carry out a meaningful search into the state of the art on the basis of some of the claims.

Claims searched completely: 1-8
Claims searched incompletely: —
Claims not searched: 9,10
Reason for the limitation of the search:

in order to art. 52(4)

| Place of search | Date of completion of the search | Examiner |
|---|---|---|
| VIENNA | 20-10-1987 | LUDWIG |

EPO Form 1505.1 03.82

European Patent Office

**PARTIAL EUROPEAN SEARCH REPORT**

EP 87304457.2

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| A | US - A - 4 468 216 (R. MUTO) <br> * Fig. 1; column 2, lines 52-55 * <br> -- | 3 | |
| D,E | EP - A1 - 0 228 762 (CHESEBROUGH POND'S) <br> * Totality * <br> ---- | 1,2,4, 7 | |
| | | | **TECHNICAL FIELDS SEARCHED (Int. Cl.4)** |